# EUROPÄISCHE PATENTSCHRIFT

(11) **EP 3 066 187 B1**
(45) Veröffentlichungstag und Bekanntmachung des Hinweises auf die Patenterteilung: **31.05.2017**
(21) Anmeldenummer: 14799660.7
(22) Anmeldetag: 31.10.2014
(51) Int. Cl.: C12M 1/107, C12M 1/00, C12M 1/34, C12M 1/02

(54) **VORRICHTUNG UND VERFAHREN ZUR TROCKNUNG VON GÄRRESTEN**
DEVICE AND METHOD FOR DRYING FERMENTATION RESIDUES
DISPOSITIF ET PROCÉDÉ DE SÉCHAGE DE RÉSIDUS DE FERMENTATION

(30) Priorität: 05.11.2013 DE 102013018464; 18.04.2014 DE 102014005667
(43) Veröffentlichungstag der Anmeldung: 14.09.2016
(73) Patentinhaber: Biogastechnik Süd GmbH, 88316 Isny im Allgäu (DE)
(72) Erfinder: MAIER, Clemens, 88316 Isny-Sommersbach (DE); MAIER, Gregor, 88316 Isny-Sommersbach (DE)
(74) Vertreter: Höchtl, Walter
(86) Internationale Anmeldenummer: PCT/DE2014/100387
(87) Internationale Veröffentlichungsnummer: WO 2015/067240

(56) Entgegenhaltungen:
- EP-A1- 1 757 562
- EP-A1- 2 275 763
- WO-A2-2008/095685
- DE-A1- 4 114 667
- DE-A1-102007 034 642
- DE-A1-102009 012 418
- DE-A1-102012 004 497
- DE-U1-202012 012 723
- KR-A- 20070 053 178

## Beschreibung

Die Erfindung betrifft eine Vorrichtung und ein Verfahren zur Trocknung von Gärresten.

Für die wachsende Anzahl von Biogasanlagen wird die Entsorgung des Gärrestes zu einer zentralen Frage. Dafür müssen praxistaugliche und wirtschaftliche Konzepte gefunden werden. Abhängig vom Ausgangsgärrest und den gewünschten Produkten kommen mechanische Separation, Trocknung, Eindampfung und Verfahren der Abwasserwirtschaft zum Einsatz. Diese einzeln oder in Kombination betriebenen Verfahren erfordern zum Teil erhebliche Investitionen und den Einsatz von thermischer und elektrischer Energie. Andererseits können Lager- und Ausbringkosten eingespart und mitunter Erlöse für die Gärrestprodukte erzielt werden. Bei Großanlagen ist eine Gärrestaufbereitung kaum zu umgehen und die Frage nach ihrer Wirtschaftlichkeit stellt sich im Zusammenhang mit der gesamten Anlage (vgl. LOOTSMA, A., T. RAUSSEN (2008): Aktuelle Verfahren zur Aufbereitung und Verwertung von Gärresten. In: Bio- und Sekundär-rohstoff-verwertung. Stofflich - energetisch III. Witzenhausen-Institut - Neues aus Forschung und Praxis. Kassel: K. Wiemer, M. Kern (Hrsg.). S. 559-576.).

Die zitierte Fundstelle gibt einen guten Überblick über den Stand der Technik der Trocknungsverfahren. Dabei werden häufig Kombinationen von Trocknungs-Methoden aus mechanischer Separation und thermischen Verfahren unter Einsatz von Band-, Trommel-, Schubwende-, Wirbelschicht-, oder Heißdampftrockner angewendet. Zur Eindampfung beziehungsweise zur Eindickung flüssiger Gärreste werden Fallstrom- und Zwangsumlaufverdampfer oder mehrstufige Verdampferkessel mit mechanischer Brüdenverdichtung verwendet. Auch Verdampfungsanlagen mit Brüdenkompression kommen zur Anwendung.

Bei auf dem Markt eingesetzten Bandtrockner-Anlagen erfolgt die Trocknung der Gärreste durch aufgeheizte Luft, die über ein großflächiges, mit Gärresten beschicktes Band geblasen wird. Das Wirkprinzip dieser Anlagen beruht im Wesentlichen auf Verdunstung. Um eine hohe Trockenleistung zu erzielen, werden hohe Luftströmungsgeschwindigkeiten erforderlich. Die Trocknung wird zusätzlich durch Erwärmung der Trockenluft unterstützt. Allerdings sind bei diesen Trocknungsverfahren große Luftleistungen notwendig die elektrisch erzeugt werden müssen. Dadurch wird der elektrische Wirkungsgrad der gesamten Biogasanlage reduziert. Außerdem ist der Trockenwirkungsgrad nicht ausreichend um 100 % der Gärreste, die in der Biogasanlage entstehen, zu verarbeiten. Darüber hinaus sind solche Anlagen nicht hermetisch dicht, so dass es zu Geruchsbelastungen kommt, wobei die Abluft zusätzlich mit Ammoniak und Feinstaub belastet wird.

Bei den meisten Verfahren zur Gärrestetrocknung müssen die Gärreste vorbehandelt werden indem sie z. Bsp. mithilfe eines Separators auf einen Trockensubstanzgehalt von 25 Prozent getrocknet werden; oder es wird ein kleiner Teil des getrockneten Gutes zugemischt. Diese Verfahrensschritte stellen zusätzlichen Aufwand dar, der den Wirkungsgrad der Trocknung verschlechtert.

Die koreanische Patentanmeldung KR 2007 0053 178 A zeigt eine zylindrische Wärmetauscher- Anordnung mit einem Gehäuse, in dem ein Niedrigtemperatur-Wärmetauscher, ein Hochtemperatur- Wärmetauscher und ein Kondensator untergebracht sind. Diese Komponenten sind durch verschiedene Rohrleitungen untereinander verbunden. Das Gehäuse selbst, das als Verdampferkessel angesehen werden könnte, weist allerdings selbst keinen Gärrestezufluss und keinen Gärresteabfluss auf und nimmt damit auch keine Gärreste auf.

Die deutsche Patentanmeldung DE 10 2007 034 642 A1 zeigt ein Verfahren und eine Anlage zur Behandlung von organisch belasteten Abfällen. Die technischen Merkmale eines mit Wärmetauschern ausgestatteten Verdampferkessels sind daraus nicht bekannt.

Aus der deutschen Offenlegungsschrift DE 41 14 667 A1 ist ein Verfahren und eine Vorrichtung zur Aufbereitung in Form von Gülle vorliegender tierischer Abfallprodukte bekannt, dass darin besteht, dass die Abfallprodukte zunächst in einem Behälter zur Bildung von Methangas gelagert werden, von dort kontinuierlich einer Zentrifuge zugeführt werden und die dort erhaltene feste Komponente anschließend getrocknet wird. Die in der Zentrifuge abgetrennte flüssige Komponente wird in einen Verdampferkessel geleitet, in dem das darin enthaltene Wasser durch Unterdruck verdampft, anschließend in einem Kondensator wieder verflüssigt und dem natürlichen Wasserkreislauf zugeführt wird. Die Abwärme des Kondensators wird mittels einer Wärmepumpe angehoben und dann dem Verdampferkessel zugeführt. Bei diesem, wie bei allen bekannten Kondensatoren erfolgt die Dampfkondensation durch herkömmliche Wärmetauscher, das heißt mit 2 getrennten Medien, was neben anderen Nachteilen einen Temperaturverlust zur Folge hat und die Wärmetauscher wegen der belasteten Dampfbrüde schnell verschmutzen

Aufgabe der Erfindung ist es daher einen Gärrestetrockner zu schaffen, der die genannten Nachteile überwindet.

Diese Aufgabe wird mit einer Vorrichtung zur Trocknung von Gärresten nach dem Patentanspruch 1 gelöst.

Eine vorteilhafte Ausbildung der Erfindung besteht darin, dass die doppelwandigen Platten aus mehreren Kreissegmenten bestehen. Dies erleichtert den Einbau und die Instandhaltung der doppelwandigen Platten.

Eine vorteilhafte Vorrichtung zur Trocknung von Gärresten besteht dabei aus mindestens zwei in Reihe angeordneten Gärrestetrocknern die jeweils über ihre Gärrestezu- und -abflüsse miteinander verbunden sind, wobei Heizvorrichtung des jeweils vorgeordneten Gärrestetrockners mit dem Dampfauslass des nachgeordneten Gärrestetrockners verbunden ist, wobei das in der Heizvorrichtung des vorgeordneten Gärrestetrockners anfallende Kondensat einem Kondensatsammelbehälter zugeführt wird.

Das anfallende Kondensat wird dabei mittels einer oder mehrerer Vakuumpumpen transportiert. Dabei ist es vorteilhaft, das aus den Heizvorrichtungen austretende Kondensat auf dem Weg zu der bzw. den Vakuumpumpen zu kühlen. Dadurch wird mehr Ammoniak im Kondensat gebunden und es kann ein größerer Volumenstrom transportiert werden oder es können Rohrleitungen mit kleinerem Durchmesser verwendet werden. Der Wirkungsgrad der Anlage erhöht sich durch diese Maßnahme deutlich.

Nach einer weiteren vorteilhaften Ausbildung der Vorrichtung zur Trocknung von Gärresten ist zwischen den Gärrestezu- und -abflüssen mindestens eine Fest-/Flüssig- Trennvorrichtung (QP) für die Gärreste (M) vorgesehen. Wenn der Anteil der festen Bestandteile der Gärreste für den Trocknungsprozess oder im Verlauf des Trocknungsprozesses zu hoch ist, kann eine mechanische Trennung der Gärreste sinnvoll sein.

Nach einer weiteren vorteilhaften Ausbildung der Vorrichtung zur Trocknung von Gärresten ist der Gärresteabfluss eines Gärrestetrockners mit dem Eingang eines Nachgärers und dessen Ausgang mit dem Gärrestezufluss des nachfolgenden Gärrestetrockners verbunden. Ein zwischen zwei Gärrestetrocknern eingefügter Nachgärer hat den Vorteil, dass bereits vor der Beschickung des Nachgärers ein großer Anteil der Brüde aus den Gärresten verdampft wird, so dass das Gärrestevolumen reduziert wird und daher der Nachgärer baulich kleiner ausgeführt werden kann. Dadurch reduziert sich zusätzlich der Wärmebedarf im Nachgärer. Darüber hinaus wird durch die vorgeschaltete Verdampfung ein besserer Aufschluss der Gärreste, i. e. des Substrats erreicht, wodurch sich höhere Gaserträge im Nachgärer ergeben.

Eine weitere vorteilhafte Ausbildung der Erfindung besteht darin, dass der Gärresteabfluss mit dem Eingang eines Nachgärers, dessen Ausgang mit einer Fest-/ Flüssig- Trennvorrichtung und dessen Flüssigstoff - Ausgang mit dem Gärrestezufluss des zweiten Gärrestetrockners verbunden ist. Je nach gewünschtem, bzw. erreichtem Trockensubstratgehalt kann es sinnvoll sein, mit insbesondere mechanischen Trennvorrichtungen, eine Trennung von flüssigen und festen Bestandteilen vorzunehmen. Diese Separation nach dem Nachgärer NG hat den Vorteil, dass aus den Gärresten/dem Substrat bereits der leicht flüchtige Ammoniak ausgetrieben ist. Dadurch ergeben sich eine wesentlich geringere Geruchsbelastung und eine Verringerung der Ammoniakemissionen. Insgesamt ergeben sich durch den besseren Substratsaufschluss höhere Gaserträge, geringere Emissionen und eine Reduzierung der Baukosten. Vorteilhaft kann, je nach gewünschtem Ergebnis, wahlweise ein Nachgärer, eine Fest- / Flüssig Trennvorrichtung, oder ein Nachgärer und eine Fest- / Flüssig Trennvorrichtung zwischen zwei Verdampfern vorgesehen werden.

Eine weitere vorteilhafte Vorrichtung zur Trocknung von Gärresten ergibt sich unter Verwendung von mindestens einem Gärrestetrockner, wobei der Anschluss des ersten Gärrestetrockners mit dem Ausgang eines Fermenters verbunden ist und der Ausgang des letzten Gärrestetrockners wieder in den Fermenter zurückgeführt wird. Durch die damit erreichbare kontinuierliche Trocknung, respektive Eindickung der Gärreste im Fermenter kann eine Volumenreduktion größer 50 % erreicht werden, so dass der Fermenter nur noch halb so groß gebaut werden muss.

Die mit dem Gärrestetrockner und den Vorrichtungen zur Trocknung von Gärresten durchführbaren Verfahren sind in den Verfahrensansprüchen definiert. Der Gärrestetrockner, die Vorrichtungen und die Verfahren werden im Folgenden anhand der Figuren näher beschrieben. Dabei zeigen:

- Figur 1: eine schematische Darstellung eines Gärrestetrockners GT in dreidimensionaler Ansicht und Seitenansicht mit teilweise aufgeschnittenem Mantel des Verdampferkessels V
- Figur 2: eine schematische Darstellung einer Vorrichtung zur Trocknung von Gärresten mit drei hintereinander geschalteten Gärrestetrocknern GT1 bis GT3
- Figur 3: eine schematische Darstellung einer Vorrichtung zur Trocknung von Gärresten mit einem Nachgärer NG und einer Trennvorrichtung QP zwischen zwei Gärrestetrocknern
- Figur 4: eine schematische Darstellung einer Vorrichtung zur Gärrestetrocknung mit zwei Gärrestetrocknern GT, die im Kreislauf mit einem Fermenter F verbunden sind

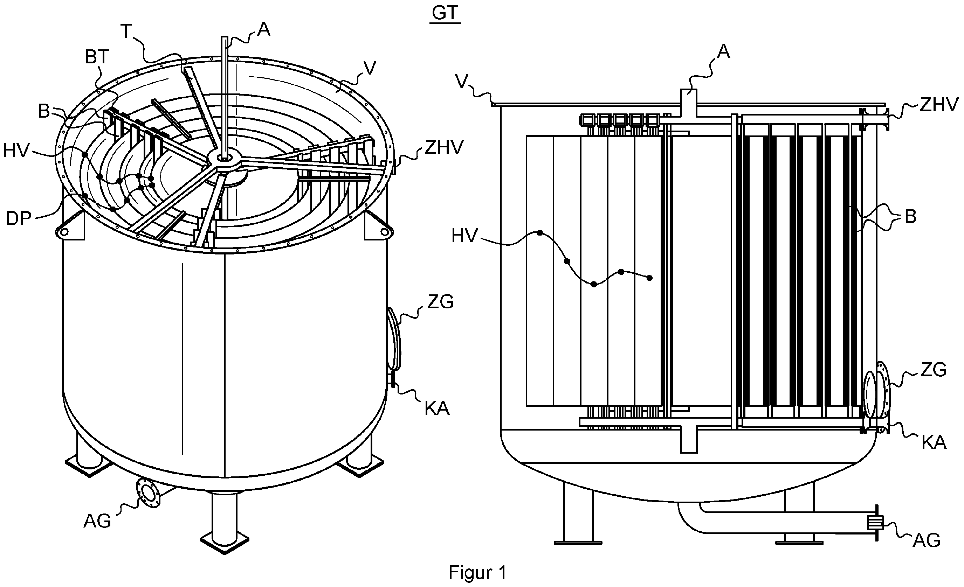

Figur 1 zeigt eine Vorrichtung zur Trocknung von Gärresten, die im Folgenden als Gärrestetrockner GT bezeichnet wird. Dieser besteht aus einem Verdampferkessel V, der in Figur 1 zwar oben offen dargestellt ist, tatsächlich aber oben geschlossen ist. Im Verdampferkessel V befinden sich konzentrisch angeordnete, doppelwandige Wärmetauscher-Platten DP, als Heizvorrichtung HV. Jede dieser doppelwandigen Platten DP ist am oberen Ende mit einer Zuleitung ZHV und am unteren Ende mit einem Ablaufrohr KA verbunden. Über die Zu- und Ableitung ZHV, KA werden Heißwasser oder Dampf durch die Platten geführt.

Es hat sich gezeigt, dass derartige Wärmetauscher direkt mit Dampf beheizt werden können. Durch den bei der Kondensation entstehenden Unterdruck (und die nachfolgend noch aufgezeigten Unterdruckhalteleitungen und Absaugung des Kondensates) wird der Dampf mit hoher Geschwindigkeit in die Wärmetauscherplatten gesaugt. Dadurch kann der Durchmesser der Dampfleitungen reduziert werden, beispielsweise von 400mm auf 150mm.

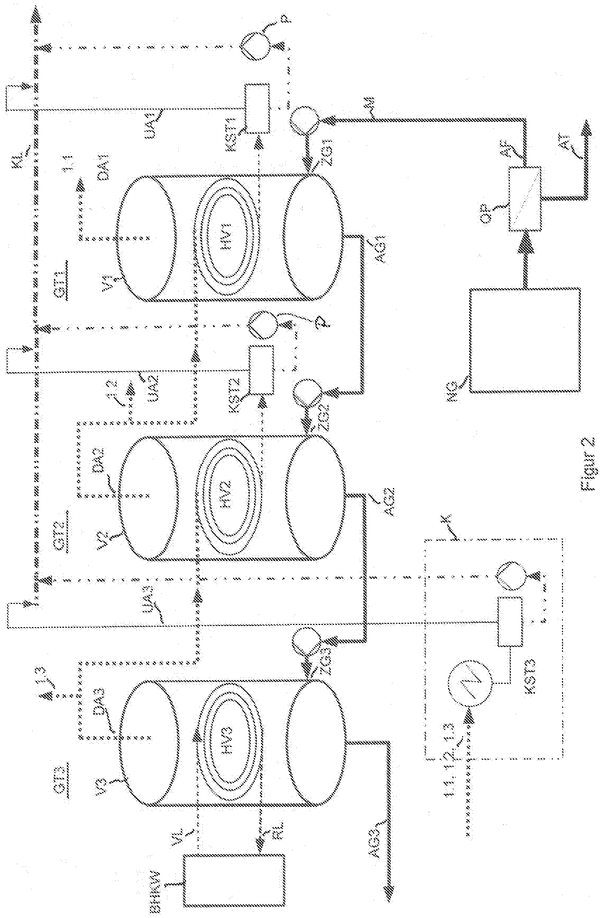

Der Verdampferkessel V wird mittels einer - in Figur 1 nicht dargestellten - Pumpe über einen Gärrestezufluss ZG mit Gärresten, die zum Beispiel aus einem Fermenter oder einem Nachgärer einer Biogasanlage, oder von einem vorgeordneten Gärrestetrockner GT (Figuren 2 bis 3) kommen, beschickt. Die Gärreste befinden sich dann zwischen den Wärmetauscherplatten DP. Die dann teilweise getrockneten Gärreste werden über den Gärresteabfluss AG ausgeschleust und wie nachfolgend gezeigt, weiterverarbeitet.

Im Verdampferkessel V ist oben und unten eine dreiarmige Haltevorrichtung T befestigt. Diese nimmt die Welle (Antriebsachse A) eines ebenfalls dreiarmigen Bürstenträger BT auf. Am Bürstenträger BT sind Bürsten B befestigt, die sich parallel zur senkrechten Achse (Antriebsachse A) des Verdampferkessels über die Länge der Wärmetauscherplatten DP der Heizvorrichtung HV erstrecken. Der Bürstenträger BT wird über die Antriebsachse A reversierend bewegt, so dass die an den drei Armen befestigten Bürsten jeweils ein Kreissegment der Platten reinigen. Dabei sind die Bürsten B so angeordnet, dass Vorder- und Rückseiten der Platten gereinigt werden.

Der Gärrestetrockner GT funktioniert somit wie ein Wärmetauscher. Durch die beheizten Platten DP werden die Gärreste erhitzt. Der dabei entstehende Dampf wird über ein - hier der Übersichtlichkeit halber nicht gezeigtes - im Deckel des Verdampferkessels V befindliches Dampfaustrittsrohr ab- und einer im Folgenden beschriebenen Weiterverwendung zugeführt. Die Heizvorrichtung HV kann mit ihrem Eingang VL und ihrem Ausgang KA an eine Heißwasserzirkulationsvorrichtung, zum Beispiel an die eines Blockheizkraftwerkes angeschlossen werden. Wird sie mit Dampf beheizt, so wird das durch den Wärmeentzug entstehende Kondensat über den Ausgang KA abgeführt.

Figur 2 zeigt eine schematische Darstellung einer Vorrichtung zur Trocknung von Gärresten mit drei hintereinander geschalteten Gärrestetrocknern GT3 bis GT1 (von links nach rechts). Die Heizvorrichtung HV3 wird über eine externe Wärmequelle, hier über Vor- und Rücklauf VL, RL der Heißwasserzirkulationsvorrichtung eines Blockheizkraftwerkes BHKW, versorgt. Der dadurch im Verdampferkessel V3 erzeugte Dampf wird über das Dampfaustrittsrohr DA der zweiten Heizvorrichtung HV2 zugeführt. Entsprechend wird die erste Heizvorrichtung HV1 mit dem Dampf aus dem Gärrestetrockner GT2 beaufschlagt. Auf diese Weise wird eine kaskadierte Versorgung der Gärrestetrockner GT3 bis GT1 mit der vom Blockheizkraftwerk BHKW originär erzeugten Wärme erreicht.

Die Gärreste M werden entgegen der Zirkulation des Dampfes nacheinander durch die Gärrestetrockner GT1 bis GT3 (in der Figur von rechts nach links) gepumpt und dabei zunehmend getrocknet. Bevor sie aus dem Nachgärer NG in den Gärrestetrockner GT1 gepumpt werden, können die Gärrest M mittels einer Fest- / Flüssig - Trennvorrichtung QP, beispielsweise einem mechanischen Schneckenseparator, in feste und mehr oder weniger flüssige Bestandteile getrennt werden. Dabei wird dann der Trockenanteil über den Ausgang AT der Trennvorrichtung QP ausgeschleust. Der Feuchteanteil wird als Gärrest M über den Ausgang AF dem Verdampferkessel V1 des Gärrestetrockners GT1 zugeführt.

Das in den Heizvorrichtungen HV1 und HV2 anfallende Kondensat wird in die Kondensatsammelbehälter KST1 und KST2 geleitet und von dort mittels Pumpen in die Kondensatleitung KL gepumpt.

Der im Verdampferkessel V1 anfallende Dampf wird über den Dampfauslass DA1 und den Leitungsanschluss 1.1 einem herkömmlichen Wärmetauscher, hier einem Dampfkondensator K (strichpunktiert umrandet), zugeführt. Das dort im Kondensatsammelbehälter KST3 anfallende Kondensat wird ebenfalls in die Leitung KL gepumpt. Die im Dampfkondensator K gewonnene Wärme kann einer weiteren Nutzung zugeführt werden.

Über die Leitungsanschlüsse 1.2 und 1.3 der Dampfauslasse DA2 und DA3 kann Dampf abgezweigt und ebenfalls dem Dampfkondensator K oder jeweils einem gesonderten Wärmetauscher zugeführt und so Warmwasser erzeugt werden. Wird beispielsweise Dampf aus dem Verdampferkessel V3 abgezweigt, kann Warmwasser mit einer Temperatur von ca. 70 °C gewonnen werden; nach dem Verdampferkessel V2 von 55 °C und nach dem Verdampferkessel V1 von 40 °C.

An jedem der Kondensatsammelbehälter KST1 bis KST3 ist eine Unterdruckleitung UA1, UA2, UA3 angeschlossen, die jeweils in die Kondensatleitung KL geführt ist. Die Kondensatleitung KL ist, wie später noch gezeigt wird, an eine Vakuumpumpe angeschlossen, wobei einerseits das Kondensat abgepumpt wird und andrerseits das gewünschte Vakuum gehalten werden kann.

Durch die Kondensation in den Wärmetauscherplatten entsteht ein Unterdruck, der sich über die Dampfauslasse in die jeweiligen Verdampferkessel V auswirkt.

Über die Unterdruckanschlüsse UA1 bis UA3 wird mit Hilfe von (hier der Übersichtlichkeit halber nicht dargestellten) Ventilen und gegebenenfalls Pumpen einerseits der Verdampfungsprozess in den Verdampfern V1 bis V3 durch Erzeugen und oder halten eines vorgebbaren Unterdrucks mit gesteuert.

Das in den Kondensatsammelbehältern KST1 bis KST3 anfallende Kondensat wird in die Kondensatleitung KL gepumpt bzw. mittels einer Vakuumpumpe abgesaugt und einer weiteren Verarbeitung, insbesondere einer Ammoniakseparation unterzogen. Für die Vakuumerzeugung kann - muss aber nicht zwangsläufig jeder Unterdruckleitung UA1 bis UA3 eine Vakuumpumpe P zugeordnet werden, sondern die verschiedenen Vakua können zum Beispiel auch über nur eine Vakuumpumpe P und entsprechende Ventile eingestellt werden. Diese Maßnahmen liegen im Können des Fachmanns und sind deshalb hier nicht weiter dargestellt und erläutert.

Das heiße, aus dem jeweiligen Kondensatablauf KA der Gärrestetrockner GT austretende Kondensat (das beispielsweise aus der Heizvorrichtung HV2 mit ca. 60 °C bis 70 °C austritt) weist noch einen mehr oder weniger großen Anteil an Dampf auf. Die standardmäßig verwendeten Vakuumpumpen sind zwar mit einer Kühlung ausgestattet. Es hat sich aber gezeigt, dass der Wirkungsgrad der Anlage erheblich verbessert werden kann, wenn das aus dem Kondensatablauf KA austretende und den Kondensatsammelbehältern KST3, KST2, KST1 zugeführte oder in diesen befindliche Kondensat zusätzlich abgekühlt wird. Dadurch erhöht sich einerseits die Leistung der Vakuumpumpen, andererseits enthält das gekühlte Kondensat weniger Kohlendioxid und es bindet sich vorteilhaft mehr Ammoniak in der Kondensatflüssigkeit. Es ist daher sinnvoll, eine zusätzliche Kühlvorrichtung zum Beispiel in oder an den Kondensatsammelbehältern KST3, KST2, KST1 oder an den Rohrleitungen zur Vakuumpumpe bzw. zu den Vakuumpumpen (falls mehrere verwendet werden), anzubringen oder / und die Vakuumpumpe(n) selbst zusätzlich zu kühlen. Eine Kühlung des Kondensats um ca. 20 °C bis 25 °C bringt bereits eine erhebliche Verbesserung des Wirkungsgrades.

Am Gärresteausgang AG3 des Verdampfers V3 fällt somit Gärrest M mit dem gewünschten Trockensubstanzgehalt von z. Bsp. 25 % an und kann in ein hier nicht dargestelltes Gärrestelager verbracht werden.

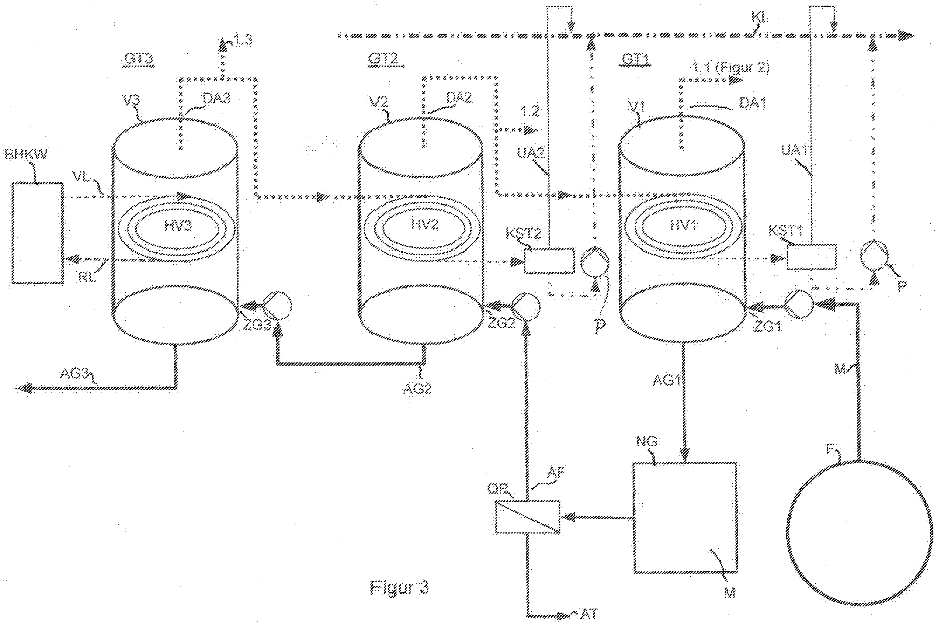

Im Ausführungsbeispiel gemäß Figur 3 ist eine schematische Darstellung einer Vorrichtung zur Trocknung von Gärresten gezeigt, bei der ein Nachgärer NG mit einer nachgeschalteten Trennvorrichtung QP zwischen zwei Gärrestetrocknern, hier zwischen den Gärrestetrocknern GT1 und GT2 angeordnet ist. Der Verdampferkessel V1 des Gärrestetrockners GT1 wird mit Gärresten aus einem Fermenter F beschickt. Im Verdampferkessel V1 kann dann bereits beispielsweise ein Drittel der Brüde aus den Gärresten M verdampft werden. Damit ergibt sich eine Volumenreduzierung der Gärreste M, die beispielsweise circa 25 % betragen kann, so dass nur noch ein um 25 % kleinerer Nachgärer NG erforderlich ist. Damit wird im Nachgärer NG entsprechend weniger Wärme benötigt. Außerdem wird durch die Verdampfung vor der Nachgärung ein besserer Substrataufschluss erreicht, was sich vorteilhaft auf die Nachgärung auswirkt.

Aus dem Nachgärer NG können die Gärreste M direkt dem zweiten Gärrestetrockner zugeführt werden, oder sie werden, wie dargestellt, einer Trennvorrichtung QP zugeführt. Diese Trennvorrichtung kann beispielsweise eine mechanische Schneckenpresse sein. Es könnte aber auch eine Zentrifuge, etc. verwendet werden. Die ausgepressten, relativ festen Bestandteile (der Trockenanteil), werden über den Ausgang AT einer weiteren Verwendung oder Verarbeitung zugeführt. Die flüssigen Anteile werden in den Verdampferkessel V2 des Gärrestetrockners GT2 eingebracht. Die Heizkaskade funktioniert entsprechend wie oben anhand von Figur 2 beschriebenen: Die dritte Heizvorrichtung HV3 des dritten Gärrestetrockners GT3 wird durch die Abwärme eines Blockheizkraftwerkes BHKW mit Heißwasser beheizt und über den in den jeweiligen Verdampferkesseln V3 bis V1 entstehenden Dampf werden jeweils die vorgeschalteten Verdampferkessel V2 und V1 über ihre Heizvorrichtungen HV2, HV1 beheizt. Der aus Figur 2 bekannte Kondensator ist der Übersichtlichkeit halber in Figur 3 nicht dargestellt, kann aber ebenfalls Bestandteil der Vorrichtung sein.

Es ist darauf hinzuweisen, dass nicht zwangsläufig eine Kombination des Nachgärers NG und einer mechanischen Trennvorrichtung QP erforderlich ist und auch nicht zwangsläufig an dieser Stelle. Die mechanische Trennvorrichtung QP oder der Nachgärer NG können auch separat zwischen je zwei Verdampfern V1 bis V3 angeordnet werden. Es kann auch sowohl nur ein Nachgärer NG oder eine mechanische Trennvorrichtung QP verwendet werden. Der Einsatz eines Nachgärers NG und / oder einer Trennvorrichtung QP in der Kaskade zwischen den Verdampferkesseln V3 bis V1 kann vom Fachmann in Abhängigkeit vom gewünschten Trocknungsgrad vorgesehen werden.

Die Trennung der festen und flüssigen Bestandteile durch die mechanische Trennvorrichtung QP nach dem Nachgärer NG hat den Vorteil, dass durch die Verdampfung im Gärrestetrockner V1 schon der leicht flüchtige Ammoniak aus dem Substrat ausgetrieben ist, und somit wesentlich weniger Geruchsbelästigungen und Ammoniakemissionen anfallen. Zusätzlich ergibt sich durch den besseren Substrataufschluss ein höherer Gasertrag; weiter ergeben sich durch die Reduzierung der Gärreste bzw. des Substratsvolumens geringere Baukosten, da der Nachgärers NG kleiner ausgeführt werden kann.

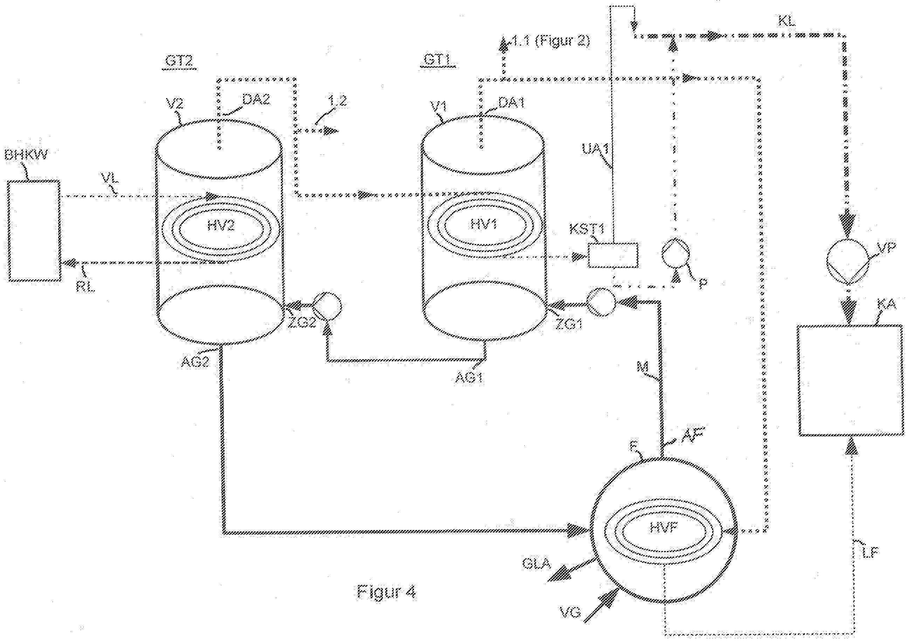

In der Darstellung gemäß Figur 4 sind zwei Gärrestetrockner GT1, GT2 im Kreislauf mit einem Fermenter F verbunden. Eine solche Anlage eignet sich besonders für die Trocknung von Gärresten mit einem hohen Flüssigkeitsanteil wie beispielsweise Gülle. Der Fermenter F wird über eine nicht näher dargestellten Güllegrube VG kontinuierlich mit Gülle beschickt. Aus dem Fermenter F durchläuft die Gülle einen Kreisprozess über die Verdampferkessel V1 und V2 und wird anschließend wieder zum Fermenter F zurückgeführt. Durch den kontinuierlichen Zulauf aus der Güllegrube VG bleibt der Trockensubstanzgehalt im Fermenter F konstant, zum Beispiel bei 10 %. Auch bei dieser Anlage erfolgt die Beheizung der Verdampferkessel V2, V1 wie oben beschrieben. Der Fermenter F kann über eine Heizvorrichtung HVF beheizt werden. Diese wird mit Dampf aus dem Dampfauslass DA1 des Verdampferkessels V1 beschickt. Auch hier besteht die Möglichkeit (wie anhand von Figur 2 beschrieben), einen Teil des Dampfes über den Anschluss 1.1 abzuzweigen und einer weiteren Heizvorrichtung zuzuführen.

Durch das Zusammenspiel von Heiztemperatur und Unterdruck können die vorbeschriebenen Anlagen optimal geregelt werden. Eine solche Regelung sei beispielhaft anhand der Anlage gemäß Figur 4 beschrieben. Die Heizvorrichtung HV2 wird über das Blockheizkraftwerk mit Heißwasser mit einer Vorlauftemperatur von 95 °C versorgt. Bei entsprechendem Unterdruck verdampft der Gärrest dann bei ca. 70 °C. Für die Heizvorrichtungen HV1 stehen somit nochmals circa 69 °C zur Verfügung. Damit kann die thermisch erzeugte Energie des Blockheizkraftwerks BHKW zweimal genutzt werden. Dies ist insbesondere bei der Vergärung von Gülle (Gülle wird im Sinne der Anmeldung als Gärrest bezeichnet) von Vorteil, da bei deren Vergärungsprozess kaum Wärme erzeugt wird. In Verdampferkessel V1 verdampft die Brüde bei entsprechendem Unterdruck bei ungefähr 60 °C. Damit steht die gesamte thermisch erzeugte Energie des Blockheizkraftwerks BHKW mit einem Temperaturniveau von knapp 60 °C weiterhin zur Beheizung des Fermenters F oder, wie beschrieben, externer Heizvorrichtungen zur Verfügung.

Über den Ausgang GLA des Fermenters F wird die eingedickte Gülle in ein Gärrestelager GL verbracht.

Stellvertretend für alle anderen beschriebenen Anlagen ist in Figur 4 eine Vakuumpumpe VP dargestellt, mit der das überschüssige Kondensat über die Kondensatleitung KL abgesaugt und einer Kondensataufbereitungsanlage KA zugeführt wird. Im Ausführungsbeispiel gemäß Figur 4 wird der Kondensataufbereitungsanlage KA zusätzlich noch das Kondensat aus dem Fermenter F Über die Leitung LF zugeführt.

Die für die Vorrichtungen notwendigen Komponenten, wie Ventile, Pumpen, Sensoren, Steuerungen, etc. sind in den Figuren nicht dargestellt. Sie gehören zum Fachwissen des Verfahrenstechnikers, der sie nach Bedarf auslegen und einsetzen kann.

## Patentansprüche

1. Vorrichtung zur Trocknung von Gärresten bestehend aus einem Gärrestetrockner (GT), dieser bestehend aus
einem Verdampferkessel (V) zur Aufnahme von Gärresten mit einem Gärrestezufluss (ZG), einem Gärresteabfluss (AG) und einem Dampfauslass (DA),
mindestens einer konzentrisch um die Mittelachse des Verdampferkessels (V) angeordneten, doppelwandigen Wärmetauscher-Platte (DP) als Heizvorrichtung (HV) mit einer Dampf- oder Heißwasserzuleitung (ZHV) und einer Kondensat- oder Heißwasserableitung (KA),
wobei sich die mindestens eine doppelwandige Wärmetauscherplatte (DP) vollständig im Inneren des Verdampferkessels (V) befindet,
wobei eine Reinigungsvorrichtung (BT, B) zur Reinigung der Außenseiten der doppelwandigen Platten (DP) vorgesehen ist und
wobei die Reinigungsvorrichtung (BT, B) Bürsten (B) umfasst, die parallel zur Achse des Verdampferkessels (V) angeordnet sind.

2. Vorrichtung zur Trocknung von Gärresten nach Anspruch 1, **dadurch gekennzeichnet, dass** die doppelwandigen Platten (DP) aus mehreren Kreissegmenten bestehen.

3. Vorrichtung zur Trocknung von Gärresten nach einem der vorstehenden Ansprüche, mit mindestens zwei in Reihe angeordneten Gärrestetrocknern (GT1, GT2, GT3), **dadurch gekennzeichnet, dass**
die Gärrestetrockner (GT1, GT2, GT3) jeweils über ihre Gärrestezu- und - abflüsse (ZG1, ZG2, ZG3, AG1, AG2, AG3) miteinander verbunden sind,
die Heizvorrichtung (HV1) des jeweils vorgeordneten Gärrestetrockners (GT1) mit dem Dampfauslass (DA2) des nachgeordneten Gärrestetrockners (GT2) verbunden ist, wobei
das in einer Heizvorrichtung (HV1) anfallendes Kondensat einem Kondensatsammelbehälter (KST) zugeführt wird.

4. Vorrichtung zur Trocknung von Gärresten nach Anspruch 3, **dadurch gekennzeichnet, dass** eine Kühlvorrichtung zur Kühlung des Kondensates vorgesehen ist.

5. Vorrichtung zur Trocknung von Gärresten nach einem der vorstehenden Ansprüche, **dadurch gekennzeichnet, dass** zwischen den Gärrestezu- und - abflüssen (ZG1, ZG2, ZG3, AG1, AG2, AG3) mindestens eine Fest-/Flüssig-Trennvorrichtung (QP) für die Gärreste (M) vorgesehen ist.

6. Vorrichtung zur Trocknung von Gärresten nach einem der vorstehenden Ansprüche, **dadurch gekennzeichnet, dass** mindestens zwischen zwei Gärrestetrocknern (GT) ein Nachgärer (NG) vorgesehen ist.

7. Vorrichtung zur Trocknung von Gärresten nach Anspruch 6, wobei dem Nachgärer (NG) eine Fest-/Flüssig- Trennvorrichtung (QP) für die Gärreste (M) nachgeordnet ist.

8. Vorrichtung zur Trocknung von Gärresten nach einem der vorstehenden Ansprüche mit mindestens einem Gärrestetrockner (GT1, GT2), **dadurch gekennzeichnet, dass** ein Fermenter (F) mit dem Gerästeausgang eines Gärrestetrockners (GT1, GT2) verbunden ist und ein Gäarresteausgang des Fermenters (F) mit dem Gärresteeingang eines Gärrestetrockners (GT1, GT2) verbunden ist.

9. Verfahren zur Trocknung von Gärresten mit mindestens zwei Gärrestetrocknern (GT) nach Anspruch 1 oder 2, wobei die Gärrestetrockner (GT) in Reihe angeordnet sind, wobei
der jeweils nachgeordnete Gärrestetrockner (GT) mit den Gärresten des vorgeordneten Gärrestetrockner (GT) beschickt wird und
die Heizvorrichtung (HV2, HV1) des jeweils vorgeordneten Gärrestetrockner (GT) mit dem Dampf bzw dem Kondensat aus dem jeweils nachgeordneten Gärrestetrockner (GT2, GT1) beheizt wird.

10. Verfahren zur Trocknung von Gärresten nach Anspruch 9, **dadurch gekennzeichnet, dass** die Gärreste (M) erst einem Nachgärer (NG) und dann dem nachfolgenden Gärrestetrockner (GT) zugeführt werden.

11. Verfahren zur Trocknung von Gärresten nach Anspruch 9 oder 10, **dadurch gekennzeichnet, dass** die Gärreste (M) im Gärrestestrom mindestens einmal in ihre Fest- und Flüssigbestandteile getrennt und die Flüssigbestandteile dem nachfolgenden Gärrestetrockner (GT) zugeführt werden.

12. Verfahren zur Trocknung von Gärresten mit mindestens einem Gärrestetrockner (GT) nach Anspruch 1 oder 2, wobei
mindestens ein Gärrestetrockner (GT) mit den Gärresten eines Fermenters (F) beschickt wird und die in den Gärrestetrocknern (GT) getrockneten Gärreste in den Fermenter zurückgeführt werden, wobei
der jeweils vorgeordnete Gärrestetrockner (GT1, GT2) mit dem Dampf bzw. dem Kondensat des nachgeordneten Gärrestetrockners (GT1, GT2) beheizt wird.

## Claims

1. Device for drying fermentation residues, consisting of a digestate dryer (GT), which comprises
- an evaporator (V) to hold the digestate with
- a digestate feeder (ZG), a digestate discharge (AG) and a steam exhaust (DA),
- at least one double-wall plate heat exchanger (DP) concentrically arranged around the centre axis of the evaporator (V) as a heating device (HV), with a steam or hot water line (ZHV) and a condensate or hot water outlet (KA),
- whereby the at least one double-wall plate heat exchanger (DP) is located completely within the evaporator (V),
- whereby a cleaning device (BT, B) is provided to clean the outside of the double-wall plates (DP), and
- whereby the cleaning device (BT, B) comprises brushes (B), which are arranged in parallel to the axis of the evaporator (V).

2. Device for drying fermentation residues according to claim 1, **characterised in that** the double-wall plates (DP) consist of several circular segments.

3. Device for drying fermentation residues according to one of the previous claims, with at least two digestate dryers (GT1, GT2, GT3) arranged in series, identified by the fact that
the digestate dryers (GT1, GT2, GT3) are interconnected via their digestate feeders and discharges (ZG1, ZG2, ZG3, AG1, AG2, AG3),
the heating device (HV1) of the respective upstream digestate dryer (GT1) is connected to the steam vent (DA2) of the downstream digestate dryer (GT2), whereby
the condensate accumulating in a heating device (HV1) is fed to a condensate tank (KST).

4. Device for drying fermentation residues according to claim 3, **characterised in that** a cooling device is provided to cool the condensate.

5. Device for drying fermentation residues as according to one of the previous claims, identified by the fact that at least one solid/liquid separator (QP) is provided for the digestate (M) between the digestate feeders and discharges (ZG1, ZG2, ZG3, AG1, AG2, AG3).

6. Device for drying fermentation residues according to one of the previous claims, **characterised in that** at least one post-fermenter (NG) is provided between two digestate dryers (GT).

7. Device for drying fermentation residues according to claim 6, whereby a solid/liquid separator (QP) for the digestate (M) is arranged downstream of the post-fermenter (NG).

8. Device for drying fermentation residues according to one of the previous claims, with at least one digestate dryer (GT1, GT2), **characterised in that** a fermenter (F) is connected to the digestate discharge of a digestate dryer (GT1, GT2), and a digestate discharge of the fermenter (F) is connected to the digestate feed of a digestate dryer (GT1, GT2).

9. Method for drying fermentation residues with at least two digestate dryers (GT) according to claims 1 and 2, whereby the digestate dryers (GT) are arranged in series, whereby
the respective downstream digestate dryer (GT) is fed with the digestate of the upstream digestate dryer (GT), and
the heating device (HV2, HV1) of the respective upstream digestate dryer (GT) is heated using the steam or condensate from the downstream digestate dryer (GT2, GT1).

10. Method for drying fermentation residues according to claim 9, **characterised in that** the digestates (M) are firstly fed to a post-fermenter (NG) and then to the downstream digestate dryer (GT).

11. Method for drying fermentation residues according to claim 9 or 10, **characterised in** fact that the digestate (M) is separated at least once into its solid and liquid parts in the digestate stream, and the liquid parts are fed to the downstream digestate dryer (GT).

12. Method for drying fermentation residues with at least one digestate dryer (GT) according to claims 1 or 2, whereby
- at least one digestate dryer (GT) is fed with the digestates of a fermenter (F), and the dried digestates in the digestate dryers (GT) are fed back to the fermenter, whereby
- the respective upstream digestate dryer (GT1, GT2) is heated using the steam or condensate from the downstream digestate dryer (GT1, GT2).

## Revendications

1. Dispositif de séchage des résidus de fermentation, comprenant un sécheur de résidus de fermentation (GT), ledit sécheur comprenant
- une chaudière de vaporisation (V) destinée à recevoir des résidus de fermentation, possédant une arrivée des résidus de fermentation (ZG), un écoulement des résidus de fermentation (AG) et une évacuation de la vapeur (DA),
- au moins une plaque d'échangeur de chaleur (DP) à double paroi agencée concentriquement autour de l'axe central de la chaudière de vaporisation (V) et servant de dispositif de chauffage (HV), comportant une conduite d'alimentation en vapeur ou en eau chaude (ZHV) et une conduite d'évacuation du condensat ou de l'eau chaude (KA),
- dans lequel au moins une plaque d'échangeur de chaleur à double paroi (DP) est entièrement agencée à l'intérieur de la chaudière de vaporisation (V),
- dans lequel un dispositif de nettoyage (BT, B) est prévu pour le nettoyage des faces extérieures des plaques à double paroi (DP) et
- dans lequel le dispositif de nettoyage (BT, B) comprend des brosses (B) agencées parallèlement à l'axe de la chaudière de vaporisation (V).

2. Dispositif de séchage des résidus de fermentation selon la revendication 1, **caractérisé en ce que** les plaques à double paroi (DP) comprennent plusieurs segments circulaires.

3. Dispositif de séchage des résidus de fermentation selon l'une des revendications ci-dessus, avec au moins deux sécheurs de résidus de fermentation (GT1, GT2, GT3) agencés en série, **caractérisé en ce que**
- les sécheurs de résidus de fermentation (GT1, GT2, GT3) sont respectivement reliés entre eux par leurs arrivées et écoulements de résidus de fermentation (ZG1, ZG2, ZG3, AG1, AG2, AG3),
- le dispositif de chauffage (HV1) du sécheur de résidus de fermentation respectivement agencé en amont (GT1) est relié à l'évacuation de vapeur (DA2) du sécheur de résidus de fermentation agencé en aval (GT2), dans lequel
- le condensat produit dans un dispositif de chauffage (HV1) est acheminé à un réservoir collecteur de condensat (KST).

4. Dispositif de séchage des résidus de fermentation selon la revendication 3, **caractérisé en ce qu'**un dispositif de refroidissement est prévu pour le refroidissement du condensat.

5. Dispositif de séchage des résidus de fermentation selon l'une des revendications ci-dessus, **caractérisé en ce qu'**au moins un dispositif de séparation solides/liquides (QP) pour les résidus de fermentation (M) est prévu entre les arrivées et écoulements de résidus de fermentation (ZG1, ZG2, ZG3, AG1, AG2, AG3).

6. Dispositif de séchage des résidus de fermentation selon l'une des revendications ci-dessus, **caractérisé en ce qu'**un post-fermenteur (NG) est au moins prévu entre deux sécheurs de résidus de fermentation (GT).

7. Dispositif de séchage des résidus de fermentation selon la revendication 6, dans lequel un dispositif de séparation solides/liquides (QP) pour les résidus de fermentation (M) est agencé en aval du post-fermenteur (NG).

8. Dispositif de séchage des résidus de fermentation selon l'une des revendications ci-dessus, avec au moins un sécheur de résidus de fermentation (GT1, GT2), **caractérisé en ce qu'**un fermenteur (F) est relié à la sortie de résidus de fermentation d'un sécheur de résidus de fermentation (GT1, GT2) et une sortie de résidus de fermentation du fermenteur (F) est reliée à l'entrée de résidus de fermentation d'un sécheur de résidus de fermentation (GT1, GT2).

9. Procédé de séchage des résidus de fermentation avec au moins deux sécheurs de résidus de fermentation (GT) selon la revendication 1 ou 2, dans lequel les sécheurs de résidus de fermentation (GT) sont agencés en série, dans lequel
le sécheur de résidus de fermentation (GT) respectivement agencé en aval est alimenté avec les résidus de fermentation du sécheur de résidus de fermentation (GT) agencé en amont et
le dispositif de chauffage (HV2, HV1) du sécheur de résidus de fermentation (GT) respectivement agencé en amont est chauffé par la vapeur ou le condensat du sécheur de résidus de fermentation (GT2, GT1) respectivement agencé en aval.

10. Procédé de séchage des résidus de fermentation selon la revendication 9, **caractérisé en ce que** les résidus de fermentation (M) sont d'abord acheminés à un post-fermenteur (NG), puis au sécheur de résidus de fermentation (GT) aval.

11. Procédé de séchage des résidus de fermentation selon la revendication 9 ou 10, **caractérisé en ce que** les résidus de fermentation (M) dans le flux de résidus de fermentation font au moins une fois l'objet d'une séparation en leurs constituants solides et liquides et **en ce que** les constituants liquides sont acheminés au sécheur de résidus de fermentation (GT) aval.

12. Procédé de séchage des résidus de fermentation avec au moins un sécheur de résidus de fermentation (GT) selon la revendication 1 ou 2, dans lequel
- au moins un sécheur de résidus de fermentation (GT) est alimenté avec les résidus de fermentation d'un fermenteur (F) et les résidus de fermentation séchés dans les sécheurs de résidus de fermentation (GT) sont réacheminés au fermenteur, dans lequel
- le sécheur de résidus de fermentation (GT1, GT2) respectivement agencé en amont est chauffé avec la vapeur ou le condensat du sécheur de résidus de fermentation (GT1, GT2) agencé en aval.
